(19) Europäisches Patentamt
European Patent Office
Office européen des brevets

(11) **EP 3 921 618 B1**

(12) **EUROPÄISCHE PATENTSCHRIFT**

(45) Veröffentlichungstag und Bekanntmachung des Hinweises auf die Patenterteilung:
**27.03.2024 Patentblatt 2024/13**

(21) Anmeldenummer: **20703433.1**

(22) Anmeldetag: **03.02.2020**

(51) Internationale Patentklassifikation (IPC):
**G01M 3/38** (2006.01)   **A61M 1/16** (2006.01)
**B01D 65/10** (2006.01)   **G01N 21/00** (2006.01)
**G01N 33/49** (2006.01)

(52) Gemeinsame Patentklassifikation (CPC):
**G01M 3/38; A61M 1/1692;** A61M 2205/15;
A61M 2205/3306; A61M 2205/70

(86) Internationale Anmeldenummer:
**PCT/EP2020/052564**

(87) Internationale Veröffentlichungsnummer:
**WO 2020/161049 (13.08.2020 Gazette 2020/33)**

(54) **SIMULATIONSMODELL ZUR SIMULATION EINER SCHLAUCHLEITUNG FÜR EINEN BLUTLECKDETEKTOR SOWIE VERFAHREN ZUM TESTEN EINES BLUTLECKDETEKTORS**

SIMULATION MODEL FOR SIMULATING A TUBE LINE FOR A BLOOD LEAKAGE DETECTOR, AND METHOD FOR TESTING A BLOOD LEAKAGE DETECTOR

MODÈLE DE SIMULATION PERMETTANT DE SIMULER UN TUYAU FLEXIBLE POUR UN DÉTECTEUR DE FUITE DE SANG ET PROCÉDÉ DE TEST D'UN DÉTECTEUR DE FUITE DE SANG

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **07.02.2019 DE 102019103028**

(43) Veröffentlichungstag der Anmeldung:
**15.12.2021 Patentblatt 2021/50**

(73) Patentinhaber: **Fresenius Medical Care Deutschland GmbH
61352 Bad Homburg (DE)**

(72) Erfinder:
• **HAHN, Lena Maria
53119 Bonn (DE)**
• **SCHRÖRS, Alexander
60389 Frankfurt a. M. (DE)**
• **CZERWONKA, Sven Marten
60437 Frankfurt a. M. (DE)**

(74) Vertreter: **Herrmann, Uwe
Lorenz Seidler Gossel
Rechtsanwälte Patentanwälte
Partnerschaft mbB
Widenmayerstraße 23
80538 München (DE)**

(56) Entgegenhaltungen:
**DE-A1-102017 001 484     US-A- 5 601 080**

**Beschreibung**

Die vorliegende Erfindung betrifft ein Simulationsmodell zur Simulation einer Schlauchleitung zur Überprüfung der Funktion eines Blutleckdetektors einer Blutbehandlungsmaschine.

Moderne Blutbehandlungsmaschinen besitzen meist verschiedene Überwachungselemente, die die Funktion der Blutbehandlungsmaschinen vor und während einer Behandlung überwachen. Eines dieser Überwachungselemente ist der Blutleckdetektor, der den Filtrat- beziehungsweise Plasmakreislauf der Blutbehandlungsmaschine auf Blutleckagen überwacht.

Eine Blutleckage liegt vor, sobald sich Blutbestandteile, die während der Behandlung nicht über den Dialysator aus dem Blut entfernt werden sollten, im Filtrat- oder Plasmakreislauf befinden. Dazu kommt es beispielsweise durch eine Ruptur der Filtermembranen im Dialysator, durch einen Filtersog oder durch zu hoch eingestellte Blutflüsse. Ein solcher Blutverlust kann unentdeckt eine große Gefährdung des Patienten darstellen.

Um dies zu vermeiden bzw. möglichst frühzeitig zu erkennen, wird mittels des Blutleckdetektors während des Betriebs der Blutbehandlungsmaschine ein optisches Messverfahren zur Erfassung einer Trübung oder Rotfärbung im Filtrat oder Plasma ausgeführt. Hierbei wird zur optischen Erfassung von Hämoglobin im Filtrat oder Plasma die Tatsache genutzt, dass im Absorptionsspektrum von Hämoglobin im sichtbaren Spektralbereich die Absorptionskurve des sauerstoffarmen und des sauerstoffreichen Hämoglobins im roten Wellenlängenbereich stark absinkt und somit viel geringer als im grünen Wellenlängenbereich zwischen 500 nm und 600 nm ist.

Der Blutleckdetektor ist meist an einer Schlauchleitung angeordnet und sendet Licht von einer definierten Wellenlänge durch den Schlauch und somit durch die in dem Schlauch fließende Flüssigkeit (beispielsweise Filtrat oder Plasma) an einen Lichtdetektor, der meist ein Phototransistor ist. Zudem weist der Blutleckdetektor meist einen zweiten Lichtdetektor bzw. Phototransistor auf, der das Licht direkt empfängt und einen Referenzwert liefert. Aus dem Absorptionsverhalten der Flüssigkeit bzw. den entsprechenden Messwerten des Phototransistors kann ermittelt werden, ob die Flüssigkeit Hämoglobin enthält.

Innerhalb eines Messzyklus des Blutleckdetektors, der meist insgesamt 400 ms dauert, liefert jeder der beiden Phototransistoren zwei Werte. Daraus ergeben sich vier Messwerte (nachstehend Messwert Rot, Messwert Grün, Referenzwert Grün und Referenzwert Rot), aus denen ein Value-Wert und ein Dimming-Wert berechnet werden können, welche das Absorptionsverhalten einer Lösung wiedergeben. Dabei sollen die Referenzwerte möglichst unabhängig von äußeren Einflüssen sein und dazu dienen, beispielsweise Alterungserscheinungen der Lichtquelle, welche meist eine LED ist, durch die Messung der direkten Abstrahlung aus den später berechneten Signalen zu eliminieren.

Bei einer normalen Blutleckage ist die Trübung im Schlauch durch Blutkörperchen noch sehr gering, und es wird das Value-Signal zur Überwachung verwendet, welches durch die nachstehende Formel berechnet wird.

$$Value = \frac{\dfrac{Messwert\ Rot * Referenzwert\ Gr\ddot{u}n}{Messwert\ Gr\ddot{u}n * Referenzwert\ Rot}}{\dfrac{Kalibriermesswert\ Rot * Kalibrierreferenzwert\ Gr\ddot{u}n}{Kalibriermesswert\ Gr\ddot{u}n * Kalibrierreferenzwert\ Rot}}$$

Dieses Signal setzt sich sowohl aus den Werten des roten Lichtes als auch aus den Werten des grünen Lichtes zusammen. Wie vorher erwähnt, kommt es durch das Hämoglobin zu einer stärkeren Absorption des grünen Lichtes, wodurch der Value-Wert dementsprechend bei einer normalen Blutleckage steigt.

Bei massiven Blutleckagen kommt es zu einer so starken Trübung im Schlauch, dass es zur Vollabsorption des Lichtes kommen kann, wodurch der Value-Wert für die Erfassung massiver Blutleckagen nicht zu gebrauchen ist, da sowohl rotes als auch grünes Licht durch die starke Trübung absorbiert werden. Daher wird für die Erkennung massiver Blutleckagen der Dimming-Wert verwendet, der sich lediglich aus den Werten des roten Lichtes gemäß der nachstehenden Formel berechnen lässt.

$$Dimming = \frac{Messwert\ Rot * Kalibrierreferenzwert\ Rot}{Referenzwert\ Rot * Kalibriermesswert\ Rot}$$

Für die Berechnung des Value-Werts sowie des Dimming-Werts werden neben den aktuellen, von den Phototransistoren gelieferten Werten auch die Kalibrierwerte verwendet. Dies sind ebenfalls vier Werte, die während einer Kalibrierung aufgenommen werden und anschließend als konstante Werte gespeichert sind. Bei der Kalibrierung wird ein mit Salzlösung, wie z.B. Natriumchlorid-Lösung (NaCl-Lösung), gefüllter Schlauch in den Blutleckdetektor eingelegt.

Zur Sicherstellung der Patientensicherheit muss auch der Blutleckdetektor einer Blutbehandlungsmaschine regelmäßig auf seine Funktion geprüft werden. Herkömmlicherweise wird der Blutleckdetektor hierbei manuell getestet, indem verschiedene definierte Testlösungen wie beispielsweise Salzlösung, Plasma, Filtrat etc. durch die Schlauchleitung geleitet werden, an welcher der Blutleckdetektor angeordnet ist.

Dieses Testverfahren ist jedoch recht aufwändig, langsam und erfordert aufgrund der benötigten Schläuche und Lösungen relativ viele Ressourcen. Zudem ist das herkömmliche Testverfahren auf bestimmte Testbedingungen, welche z.B. durch Salzlösung, Plasma oder Filtrat in der Schlauchleitung erzeugt werden, begrenzt. DE 10 2017 001484 A1 betrifft ein Verfahren und eine Anordnung zum Kalibrieren von Vorrichtungen zur Erkennung von Blut oder Blutbestandteilen in einer Flüssigkeit, insbesondere Dialysierflüssigkeit. US 5 601 080 A betrifft ein spektrophotometrisches Verfahren und eine Vorrichtung, die insbesondere für die Online-Überwachung und - Kontrolle von Blutparametern geeignet sind.

Der vorliegenden Erfindung liegt somit die Aufgabe zugrunde, die Nachteile des Stands der Technik abzumildern oder zu beheben. Insbesondere liegt der vorliegenden Erfindung die Aufgabe zugrunde, ein schnelleres und ressourcenschonenderes Testverfahren sowie eine Vorrichtung zur Durchführung eines solchen Testverfahrens bereitzustellen.

Diese Aufgabe wird durch das Simulationsmodell gemäß Anspruch 1 sowie das Verfahren nach Anspruch 11 gelöst. Ein weiterer Aspekt der vorliegenden Erfindung betrifft eine Blutbehandlungsmaschine, insbesondere ein Dialysegerät, mit einem Simulationsmodell gemäß Anspruch 1.

Ein erfindungsgemäßes Simulationsmodell ermöglicht es, dass mittels eines einzigen Bauteils verschiedene Testbedingungen wie z.B. Filtrat in der Schlauchleitung oder Salzlösung in der Schlauchleitung simuliert werden können, ohne das tatsächliche Lösungen oder Schlauchleitungen zum Einsatz kommen. Mit einem derartigen Simulationsmodell kann die Funktion des Blutleckdetektors automatisiert und / oder (voll-) automatisch überwacht werden.

Ein erfindungsgemäßes Simulationsmodell zur Simulation einer Schlauchleitung weist auf: mindestens einem hohlzylindrischen Abschnitt, welcher dazu ausgelegt ist, in einer Schlauchaufnahme eines Blutleckdetektors, vorzugsweise eines Blutleckdetektors einer Blutbehandlungsmaschine, angeordnet zu werden, wobei an den axialen Enden des hohlzylindrischen Abschnitts jeweils eine Aufnahme für jeweils eine Lichtquelle vorgesehen ist, mittels der Licht in den hohlzylindrischen Abschnitt eingeleitet werden kann, und mindestens einem Lichtlenkungselement, welches in dem hohlzylindrischen Abschnitt angeordnet ist und dazu ausgelegt ist, in den hohlzylindrischen Abschnitt eingeleitetes Licht derart abzulenken, dass das Licht durch mindestens eine Öffnung in einer radialen Außenseite des hohlzylindrischen Abschnitts aus dem hohlzylindrischen Abschnitt nach außen gelangt. In den Aufnahmen an den axialen Enden des hohlzylindrischen Abschnitts jeweils eine Lichtquelle angeordnet ist, welche vorzugsweise rotes und / oder grünes Licht in den hohlzylindrischen Abschnitt einleitet.

Der hohlzylindrische Abschnitt des Simulationsmodells bildet hierbei vorzugsweise eine Schlauchleitung nach, welche herkömmlicherweise in die Schlauchaufnahme des Blutleckdetektors eingelegt würde.

Mittels der Lichtquellen kann Licht mit einem vorbestimmten Rot- und / oder Grünanteil in den hohlzylindrischen Abschnitt eingeleitet werden, sodass das Absorptionsverhalten einer Lösung wie beispielsweise einer Salzlösung oder von Plasma oder einer beliebigen anderen Lösung bzw. eines Fluids nachgebildet werden kann. Durch die Einstellung der Mischung des Lichts können verschiedene Lösungen simuliert / nachgebildet werden.

Die Lichtquellen können, vorzugsweise durch Verpressung oder Verklebung, dauerhaft in den Aufnahmen des Simulationsmodells verbaut sein. Alternativ dazu können die Lichtquellen auch an einer Blutbehandlungsmaschine angeordnet sein und / oder der Blutbehandlungsmaschine zugeordnet sein und nur in die Aufnahmen des Simulationsmodells eingeführt werden, wenn das Simulationsmodell beispielsweise zur Überprüfung der Funktion eines Blutleckdetektors der Blutbehandlungsmaschine zum Einsatz kommt.

Es hat sich als vorteilhaft erwiesen, wenn das Lichtlenkungselement ein Spiegel ist, welcher derart angeordnet ist, dass er das eingeleitete Licht bricht und um ca. 90 ° ablenkt. Durch das Lichtlenkungselement wird das von den Lichtquellen eingebrachte Licht durch die mindestens eine Öffnung in dem hohlzylindrischen Abschnitt des Simulationsmodells zu mindestens einem Lichtdetektor, wie beispielsweise einem Phototransistor, umgelenkt.

Weiterhin hat es sich als vorteilhaft erwiesen, wenn in der radialen Außenseite des hohlzylindrischen Abschnitts zwei vorzugsweise einander gegenüberliegende Öffnungen vorgesehen sind, zwischen denen vorzugsweise das mindestens eine Lichtlenkungselement angeordnet ist.

Nicht gemäß der beanspruchten Erfindung, kann jedoch auch nur eine Lichtquelle an bzw. bei dem Simulationsmodell vorgesehen sein. Zudem kann die Lichtquelle Licht einer beliebigen anderen Wellenlänge abgeben, beispielsweise im sichtbaren oder nicht-sichtbaren Bereich, im Infrarotbereich oder im UV-Bereich.

Vorzugsweise sind die Lichtquellen LEDs, welche in der Aufnahme vorzugsweise durch Presspassung und / oder Verklebung fest verankert sind.

Um die Benutzerfreundlichkeit des Simulationsmodells zu erhöhen und eine sichere Verankerung des Simulationsmodells an einem Blutleckdetektor sicherzustellen, kann das Simulationsmodell zusätzlich mit einem Griffabschnitt ausgestattet sein, welcher dafür vorgesehen ist, ein Ergreifen des Simulationsmodells durch einen Benutzer zu erleichtern und sich vorzugsweise bogenförmig oder bügelförmig von einem ersten axialen Endabschnitt des hohlzylindrischen Abschnitts zu einem zweiten axialen Endabschnitt des hohlzylindrischen Abschnitts erstreckt.

Weiterhin hat es sich als vorteilhaft erwiesen, wenn das Simulationsmodell aus einem für in den hohlzylindrischen Abschnitt eingeleitetes Licht sowie für Umgebungslicht opaken / undurchlässigen Material, vorzugsweise aus Metall und / oder Kunststoff, gefertigt ist. Das Simulationsmodell kann mittels eines additiven oder subtraktiven 3D-Druckverfahrens gefertigt sein. Vorzugsweise ist das Material des Simulationsmodells dunkel, beispielsweise schwarz, gefärbt. Hierdurch wird eine besonders gute Abschirmung des Innenraums des hohlzylindrischen Abschnitts von störendem Umgebungslicht erreicht.

Das Simulationsmodell kann weiterhin zusätzlich eine Steuerungsvorrichtung aufweisen, welche die Lichtquellen des Simulationsmodells steuert oder regelt. Beispielsweise kann mittels der Steuerungsvorrichtung die Mischung (beispielsweise der Grünanteil und / oder Rotanteil) des von der mindestens einen Lichtquelle oder den Lichtquellen ausgesandten Lichts variiert bzw. eingestellt werden.

Ein weiterer Aspekt der Erfindung betrifft eine Blutbehandlungsmaschine mit einem erfindungsgemäßen Simulationsmodell und einem Blutleckdetektor, welcher mindestens einen Lichtdetektor aufweist, wobei die mindestens eine Öffnung in der radialen Außenseite des hohlzylindrischen Abschnitts des Simulationsmodells und der mindestens eine Lichtdetektor des Blutleckdetektors relativ zueinander derart angeordnet sind, dass in den hohlzylindrischen Abschnitt eingeleitetes und von dem Lichtlenkungselement abgelenktes Licht durch die mindestens eine Öffnung zu dem mindestens einen Lichtdetektor gelenkt wird. Vorzugsweise ist der Lichtdetektor ein Phototransistor.

Das Simulationsmodell kann lösbar oder unlösbar mit der Blutbehandlungsmaschine verbunden sein.

Ein anderer Aspekt der Erfindung betrifft ein Verfahren zur Überprüfung der Funktion eines Blutleckdetektors einer Blutbehandlungsmaschine und / oder der Funktion einer BLutbehandlungsmaschine, bei dem ein Simulationsmodell an dem Blutleckdetektor angeordnet wird, wobei vorzugsweise der hohlzylindrische Abschnitt des Simulationsmodells in einer Schlauchaufnahme des Blutleckdetektors angeordnet wird.

Mittels der Lichtquellen des Simulationsmodells wird Licht mit einem vorbestimmten Rot- und / oder Grünanteil in den hohlzylindrischen Abschnitt des Simulationsmodells eingeleitet, wobei der Rot- und / oder Grünanteil des von der Lichtquelle eingeleiteten Lichts derart eingestellt ist, dass er dem Rot- und / oder Grünanteil von Licht entspricht, welches reflektiert wird, wenn, während in einem in der Schlauchaufnahme angeordneten Schlauch entweder kein Fluid oder ein bestimmtes Fluid (wie z.B. Salzlösung, Plasma oder Filtrat) fließt, Licht von einer vorbestimmten Wellenlänge in oder durch den Schlauch und / oder das Fluid gesandt wird. Die Mischung des Lichts bildet somit das Absorptionsverhalten einer bestimmten Lösung nach bzw. simuliert dieses.

Vorzugsweise wird der Rot- und / oder Grünanteil des von der Lichtquelle eingeleiteten Lichts derart eingestellt, dass er dem Rot- und / oder Grünanteil von Licht entspricht, welches reflektiert wird, wenn, während in dem in der Schlauchaufnahme angeordneten Schlauch Salzlösung oder Plasma oder Dialysierflüssigkeit oder Plasma mit einem bestimmten Blutanteil fließt und Licht von einer vorbestimmten Wellenlänge in oder durch den Schlauch und / oder das Fluid gesandt wird. Mittels des Simulationsmodells können somit die herkömmlichen Testbedingungen (Salzlösung, Plasma, Dialysierflüssigkeit, Plasma mit einem bestimmten Blutanteil) nachgestellt werden. Mittels des Simulationsmodells bzw. -verfahrens können jedoch auch beliebige andere Testbedigungen simuliert werden, da Licht einer beliebigen Wellenlänge und / oder Zusammensetzung dazu verwendet werden kann, ein beliebiges Absorptionsverhalten zu simulieren.

Für verschiedene vordefinierte Zustände bzw. Testbedingungen sind die erwarteten Dimming- und Value-Werte bekannt. Diese Zustände sind beispielsweise: Im Blutleckdetektor eingelegter mit NaCl-Lösung gefüllter Schlauch, normales Blutleck beispielsweise im Plasma, massives Blutleck beispielsweise im Plasma, kein Schlauch im Blutleckdetektor eingelegt, leerer Schlauch im Blutleckdetektor eingelegt, Hämolyse beispielsweise im Plasma, Verletzung der gültigen Wertebereiche, Luftblasen im Schlauch, Fremdlichteinwirkung auf den Blutleckdetektor, Simulation des T0-Tests (Anfangstest bei Inbetriebnahme einer Blutbehandlungsmaschine).

Insbesondere werden gemäß den nachstehenden Formeln die einen bestimmten Zustand kennzeichnenden Messwerte des roten und grünen Lichts (nachstehend Messwert Rot und Messwert Grün) berechnet, die dann mithilfe eines Digital-Analog-Converters durch die Lichtquellen bzw. LEDs im Simulationsmodel eingestellt werden und zu den einem bestimmten Zustand zugeordneten Value- und Dimming-Werten führen.

$$Messwert\ Rot = \frac{Dimming * Referenzwert\ Rot * Kalibriermesswert\ Rot}{Kalibrierreferenzwert\ Rot}$$

$$Messwert\ Grün = \frac{Messwert\ Rot * Referenzwert\ Grün * Kalibriermesswert\ Grün * Kalibrierreferenzwert\ Rot}{Kalibriermesswert\ Rot * Referenzwert\ Rot * Kalibriernesswert\ Grün * Value}$$

Auf diese Weise lassen sich die Zustände bzw. Testbedingungen in dem Simulationsmodell simulieren.

Die Blutbehandlungsmaschine kann weiterhin zusätzlich eine Steuerungsvorrichtung aufweisen, welche die Lichtquellen des Simulationsmodells steuert oder regelt. Beispielsweise kann mittels der Steuerungsvorrichtung die Mischung (beispielswese der Grünanteil und / oder Rotanteil) des von den Lichtquellen ausgesandten Lichts variiert bzw. eingestellt werden.

Ein erfindungsgemäßes Verfahren kann die folgenden Schritte aufweisen: Anschließen eines erfindungsgemäßen Simulationsmodells an eine Steuervorrichtung, insbesondere wenn das Simulationsmodell selbst keine Steuervorrichtung aufweist; gegebenenfalls Anschließen des Simulationsmodells an ein Test-Steuergerät, welches zur Durchführung eines Tests ausgelegt ist und mittels dessen beispielswese ein Benutzer eine bestimmte Testbedingung vorgeben kann; Anordnen des Simulationsmodells an bzw. in einem Blutleckdetektor; Synchronisieren der Frequenz des Leuchtzyklus der Lichtquellen (meist LEDs) mit der Aufnahmefrequenz bzw. Messfrequenz des Blutleckdetektors, vorzugsweise durch optische Taktermittlung oder Auslesen einer DB-Variable; Aussenden von Licht von den Lichtquellen (meist LEDs), welches zu dem mindestens einen Lichtdetektor (oder Lichtdetektoren, meist Phototransistoren) geleitet wird, wobei die gewünschten Messwerte bzw. Referenzwerte erreicht werden, um die vorgegeben Testbedingung nachzubilden bzw. zu simulieren.

DB-Variablen sind Variablen, die über einen CAN-Adapter von der Blutbehandlungsmaschine auf einen Windows-Computer übertragen werden können. Sie können manipuliert werden, indem ihnen über ein Skript oder über das sogenannte DB-Tool Werte zugewiesen werden, um der Blutbehandlungsmaschine Zustände vorzugaukeln, die in Realität gar nicht anliegen.

Ein erfindungsgemäßes Verfahren kann dazu eingesetzt werden, den Blutleckdetektor selbst auf seine Funktion zu testen oder die Blutbehandlungsmaschine auf die Reaktionen der Blutbehandlungsmaschine auf verschiedene (Alarm-)Zustände in dem Blutleckdetektor zu überprüfen.

An dieser Stelle wird darauf hingewiesen, dass die Begriffe "ein" und "eine" nicht zwingend auf genau eines der Elemente verweisen, wenngleich dies eine mögliche Ausführung darstellt, sondern auch eine Mehrzahl der Elemente bezeichnen können. Ebenso schließt die Verwendung des Plurals auch das Vorhandensein des fraglichen Elementes in der Einzahl ein und umgekehrt umfasst der Singular auch mehrere der fraglichen Elemente.

Weitere Einzelheiten und Vorteile der vorliegenden Erfindung werden anhand eines in der Zeichnung dargestellten Ausführungsbeispiels näher erläutert.

Es zeigen:

Fig. 1:    eine schematische Ansicht, welche die Funktionsweise eines Blutleckdetektors verdeutlicht;

Fig. 2:    eine Schnittansicht eines Blutleckdetektors;

Fig. 3:    eine schematische Ansicht, welche die Funktionsweise eines erfindungsgemäßen Simulationsmodells verdeutlicht;

Fig. 4:    eine Draufsicht der Vorderseite eines Simulationsmodells gemäß einer bevorzugten Ausführungsform der Erfindung;

Fig. 5:    eine Draufsicht der Rückseite eines Simulationsmodells gemäß einer bevorzugten Ausführungsform der Erfindung;

Fig. 6:    das Simulationsmodell aus den Figuren 4 und 5 im an einem Blutleckdetektor angeordneten Zustand; und

Fig. 7:    das Simulationsmodell aus den Figuren 4 und 5 im an einem Blutleckdetektor angeordneten Zustand, wobei der Blutleckdetektor geschlossen ist.

In den Figuren bezeichnen gleiche Bezugszeichen gleiche oder funktionsgleiche Bauteile.

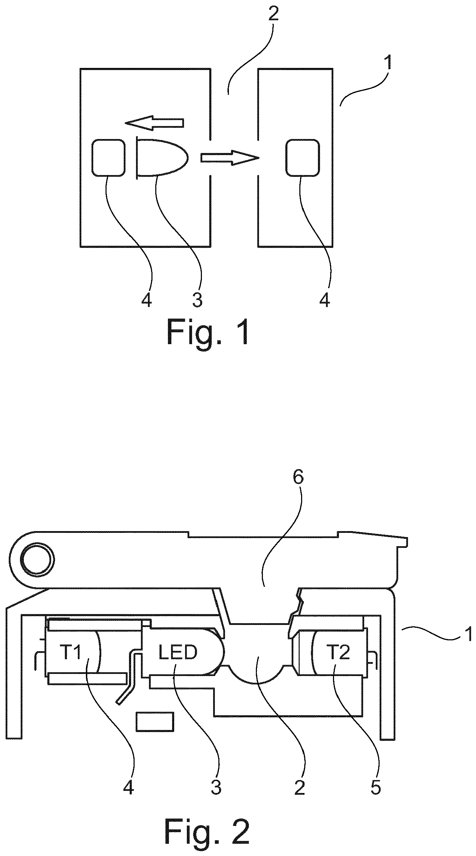

Wie in Fig. 1 gezeigt, weist ein Blutleckdetektor 1 eine Schlauchaufnahme 2 auf, in welcher ein Schlauch bzw. eine Schlauchleitung angeordnet werden kann.

Der Blutleckdetektor 1 weist zudem eine LED 3 sowie zwei Phototransistoren 4 und 5 auf. Das von der LED 3 abgestrahlte Licht ist in der Figur 1 durch Pfeile wiedergegeben.

Licht wird von der LED 3 durch die Schlauchaufnahme 2 und einen gegebenenfalls in dieser angeordneten Schlauch zu dem Phototransistor 4 gesandt. Der Phototransistor 4 erfasst einen entsprechenden Messwert. Zudem wird Licht von der LED 3 direkt zu dem Phototransistor 3 gesandt. Der Phototransistor 3 erfasst einen entsprechenden Referenzwert.

Wie aus der Schnittansicht eines Blutleckdetektors in Fig. 2 erkennbar, weist der Blutleckdetektor 1 weiterhin eine Tür bzw. Klappe 6 auf, welche nach dem Einlegen eines Schlauchs in die Schlauchaufnahme 2 geschlossen werden kann, sodass der Schlauch fest in der Schlauchaufnahme 2 gehalten ist.

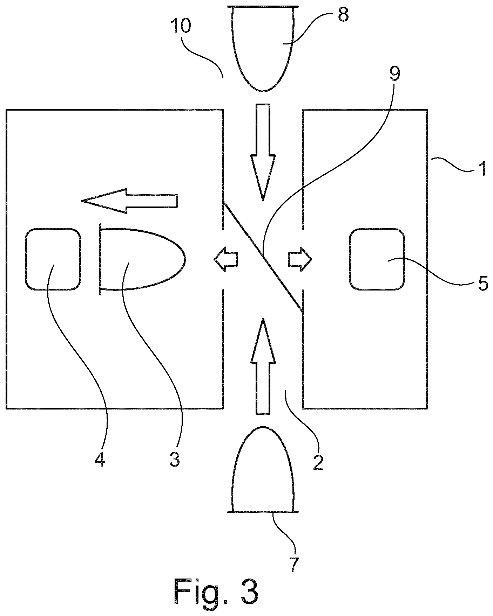

Fig. 3 zeigt schematisch ein in einem Blutleckdetektor 1 angeordnetes Simulationsmodell 10, welches zwei LEDs 7 und 8 sowie einen Spiegel 9 aufweist. In der Schlauchaufnahme 2 ist der hier nicht gezeigte hohlzylindrische Abschnitt des Simulationsmodells angeordnet.

Von den LEDs 7 und 8 in den hohlzylindrischen Abschnitt des Simulationsmodells eingeleitetes Licht (dargestellt durch die Pfeile) wird von dem Spiegel 9 im Wesentlichen rechtwinklig abgelenkt und zu den Phototransistoren 4 und 5 des Blutleckdetektors gelenkt.

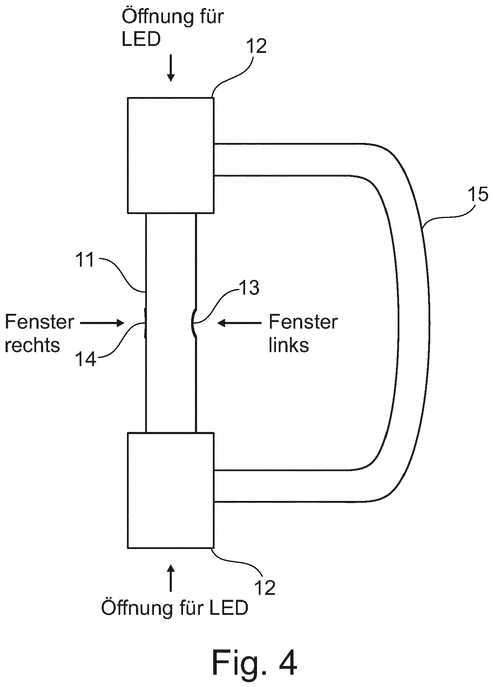

Fig. 4 zeigt eine Draufsicht der Vorderseite eines Simulationsmodells 10. Das Simulationsmodell weist einen hohlzylindrischen Abschnitt 11 auf, an dessen axialen Enden jeweils Aufnahmen 12 zur Aufnahme der LEDs 7 und 8 angeordnet sind. In der radialen Außenseite des hohlzylindrischen Abschnitts sind zwei einander gegenüberliegende Öffnungen 13, 14 ausgebildet, durch die Licht aus dem hohlzylindrischen Abschnitt 11 nach außen gelangen kann.

Das Simulationsmodell 10 weist weiterhin einen bügelförmigen Griffabschnitt 15 auf, welcher sich von einer Aufnahme 12 zu der anderen Aufnahme 12 erstreckt.

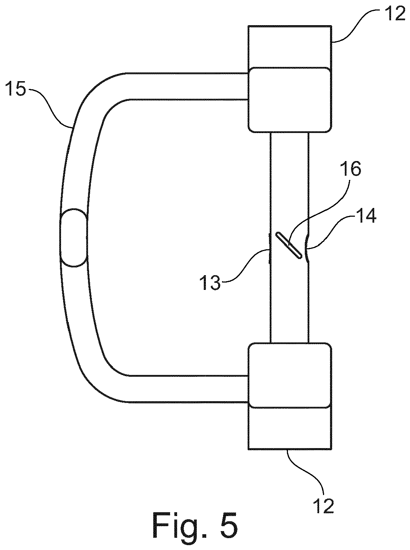

In Fig. 5 ist der Spiegel 16 zu erkennen, der zwischen der Öffnung 13 und der Öffnung 14 derart angeordnet ist, dass Licht im Wesentlichen um 90° abgelenkt und durch die Öffnungen 13 bzw. 14 gelenkt wird.

In der gezeigten Ausführungsform ist das Simulationsmodell 10 durch ein additives 3D-Druckverfahren einstückig aus einem schwarzen Material gefertigt.

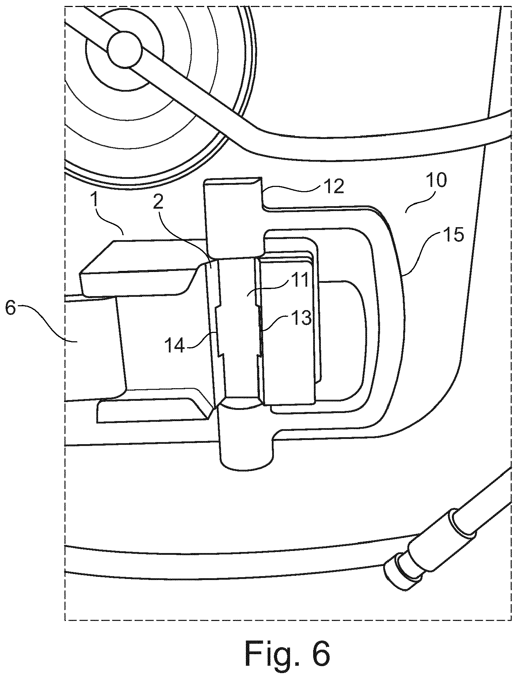

Fig. 6 zeigt das Simulationsmodell 10, welches an dem Blutleckdetektor 1 angeordnet ist. Die Tür 6 des Blutleckdetektors 1 ist geöffnet, sodass der in der Schlauchaufnahme 2 angeordnete hohlzylindrische Abschnitt 11 zu erkennen ist.

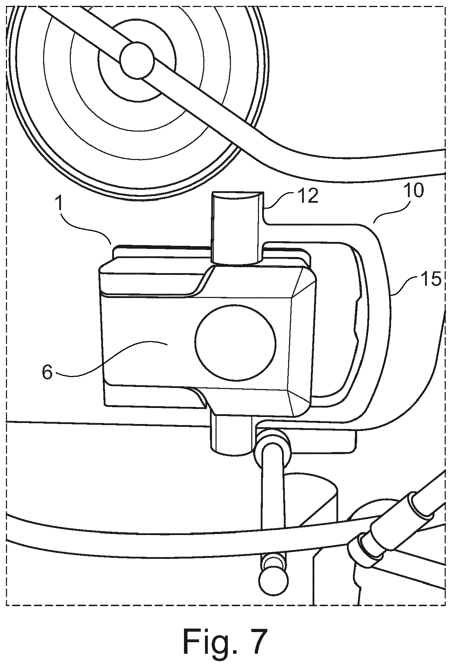

Bei der Darstellung in Fig. 7 ist die Tür 6 des Blutleckdetektors 1 geschlossen, sodass der in der Schlauchaufnahme 2 angeordnete hohlzylindrische Abschnitt 11 in dem Blutleckdetektor fest gehalten ist.

**Patentansprüche**

1. Simulationsmodell (10) zur Simulation einer Schlauchleitung, mit mindestens einem hohlzylindrischen Abschnitt (11), welcher dazu ausgelegt ist, in einer Schlauchaufnahme (2) eines Blutleckdetektors (1), vorzugsweise eines Blutleckdetektors (1) einer Blutbehandlungsmaschine, angeordnet zu werden, wobei an den axialen Enden des hohlzylindrischen Abschnitts (11) jeweils eine Aufnahme (12) für jeweils eine Lichtquelle vorgesehen ist, mittels der Licht in den hohlzylindrischen Abschnitt (11) eingeleitet werden kann, und mindestens einem Lichtlenkungselement, welches in dem hohlzylindrischen Abschnitt (11) angeordnet ist und dazu ausgelegt ist, in den hohlzylindrischen Abschnitt (11) eingeleitetes Licht derart abzulenken, dass das Licht durch mindestens eine Öffnung (13, 14) in einer radialen Außenseite des hohlzylindrischen Abschnitts (11) aus dem hohlzylindrischen Abschnitt (11) nach außen gelangt, **dadurch gekennzeichnet, dass** in den Aufnahmen (12) an den axialen Enden des hohlzylindrischen Abschnitts (11) jeweils eine Lichtquelle angeordnet ist, welche vorzugsweise rotes und / oder grünes Licht in den hohlzylindrischen Abschnitt (11) einleitet.

2. Simulationsmodell (10) nach Anspruch 1, **dadurch gekennzeichnet, dass** das Lichtlenkungselement ein Spiegel (16) ist, welcher derart angeordnet ist, dass er das eingeleitete Licht im Wesentlichen um 90 ° ablenkt.

3. Simulationsmodell (10) nach einem der vorhergehenden Ansprüche, **dadurch gekennzeichnet, dass** in der radialen Außenseite des hohlzylindrischen Abschnitts (11) zwei vorzugsweise einander gegenüberliegende Öffnungen (13, 14) vorgesehen sind, zwischen denen vorzugsweise das mindestens eine Lichtlenkungselement angeordnet ist.

4. Simulationsmodell (10) nach Anspruch 1, **dadurch gekennzeichnet, dass** die Lichtquelle eine LED (7, 8) ist, welche in der Aufnahme (12) vorzugsweise durch Presspassung und / oder Verklebung fest verankert ist.

5. Simulationsmodell (10) nach einem der vorhergehenden Ansprüche, zusätzlich mit einem Griffabschnitt (15), welcher dafür vorgesehen ist, ein Ergreifen des Simulationsmodells (10) durch einen Benutzer zu erleichtern und sich vorzugsweise bogenförmig oder bügelförmig von einem ersten axialen Endabschnitt des hohlzylindrischen Abschnitts (11) zu einem zweiten axialen Endabschnitt des hohlzylindrischen Abschnitts (11) erstreckt.

6. Simulationsmodell (10) nach einem der vorhergehenden Ansprüche, **dadurch gekennzeichnet, dass** das Simulationsmodell (10) aus einem für in den hohlzylindrischen Abschnitt (11) eingeleitetes Licht sowie für Umgebungslicht opaken Material, vorzugsweise aus Metall und / oder Kunststoff, gefertigt ist.

7. Blutbehandlungsmaschine mit einem Simulationsmodell (10) nach einem der vorhergehenden Ansprüche und einem Blutleckdetektor (1), welcher mindestens einen Lichtdetektor aufweist, **dadurch gekennzeichnet, dass** die mindestens eine Öffnung (13, 14) in der radialen Außenseite des hohlzylindrischen Abschnitts (11) des Simulationsmodells (10) und der mindestens eine Lichtdetektor des Blutleckdetektors (1) derart angeordnet sind, dass von dem Lichtlenkungselement abgelenktes in den hohlzylindrischen Abschnitt (11) eingeleitetes Licht durch die mindestens eine Öffnung (13, 14) zu dem mindestens einen Lichtdetektor gelenkt wird.

8.  Blutbehandlungsmaschine nach Anspruch 7, **dadurch gekennzeichnet, dass** das Simulationsmodell (10) lösbar oder unlösbar mit der Blutbehandlungsmaschine verbunden ist.

9.  Blutbehandlungsmaschine nach Anspruch 7 oder 8, **dadurch gekennzeichnet, dass** die Blutbehandlungsmaschine eine Steuerungsvorrichtung aufweist, die dazu ausgebildet ist, die Lichtquellen eines Simulationsmodells (10) nach einem der Ansprüche 1 bis 6 zu steuern oder zu regeln.

10. Blutbehandlungsmaschine nach Anspruch 9, **dadurch gekennzeichnet, dass** die Steuerungsvorrichtung weiterhin dazu ausgelegt ist, eine Frequenz des Leuchtzyklus der Lichtquellen des Simulationsmodells (10) nach einem der Ansprüche 1 bis 6 mit der Aufnahmefrequenz oder Messfrequenz des Blutleckdetektors (1) zu synchronisieren.

11. Verfahren zur Überprüfung der Funktion eines Blutleckdetektors (1) einer Blutbehandlungsmaschine, mit dem Schritt: Anordnen eines Simulationsmodells (10) nach einem der Ansprüche 1 bis 6 an dem Blutleckdetektor (1), vorzugsweise Anordnen des hohlzylindrischen Abschnitts (11) des Simulationsmodells (10) in einer Schlauchaufnahme (2) des Blutleckdetektors (1).

12. Verfahren nach Anspruch 11, weiterhin mit dem Schritt: Einleiten von Licht mit einem vorbestimmten Rot- und / oder Grünanteil in den hohlzylindrischen Abschnitt (11) des Simulationsmodells (10) mittels mindestens einer Lichtquelle, wobei der Rot- und / oder Grünanteil des von der Lichtquelle eingeleiteten Lichts derart eingestellt ist, dass er dem Rot- und / oder Grünanteil von Licht entspricht, welches reflektiert wird, wenn, während in einem in der Schlauchaufnahme (2) angeordneten Schlauch entweder kein oder ein bestimmtes Fluid fließt, Licht von einer vorbestimmten Wellenlänge in oder durch den Schlauch und / oder das Fluid gesandt wird.

13. Verfahren nach Anspruch 12, **dadurch gekennzeichnet, dass** der Rot- und / oder Grünanteil des von der Lichtquelle eingeleiteten Lichts derart eingestellt ist, dass er dem Rot- und / oder Grünanteil von Licht entspricht, welches reflektiert wird, wenn, während in dem in der Schlauchaufnahme (2) angeordneten Schlauch Salzlösung oder Plasma oder Dialysierflüssigkeit oder Plasma mit einem bestimmten Blutanteil fließt und Licht von einer vorbestimmten Wellenlänge in oder durch den Schlauch und / oder das Fluid gesandt wird.

## Claims

1.  A simulation model (10) for simulating a tube line comprising at least one hollow cylindrical section (11) that is configured to be arranged in a tube receiver (2) of a blood leak detector (1), preferably of a blood leak detector (1) of a blood treatment machine, wherein a respective receiver (12) for a respective light source is provided at the axial ends of the hollow cylindrical section (11), by means of which light source light can be introduced into the hollow cylindrical section (11); and at least one light guidance element that is arranged in the hollow cylindrical section (11) and that is configured to deflect light introduced into the hollow cylindrical section (11) such that the light moves outwardly out of the hollow cylindrical section (11) through at least one opening (13, 14) in a radial outer side of the hollow cylindrical section (11), **characterized in that** a respective light source that preferably introduces red and/or green light into the hollow cylindrical section (11) is arranged in the receivers (12) at the axial ends of the hollow cylindrical section (11).

2.  A simulation model (10) in accordance with claim 1, **characterized in that** the light guidance element is a mirror (16) that is arranged such that it substantially deflects the introduced light by 90°.

3.  A simulation model (10) in accordance with one of the preceding claims, **characterized in that** two openings (13, 14) are provided in the radial outer side of the hollow cylindrical section (11) that are preferably disposed opposite one another and between which the at least one light guidance element is preferably arranged.

4.  A simulation model (10) in accordance with claim 1, **characterized in that** the light source is an LED (7, 8) that is firmly anchored in the receiver (12), preferably by a press fit and/or by an adhesive bond.

5.  A simulation model (10) in accordance with one of the preceding claims, additionally comprising a handle section (15) that is provided to facilitate a gripping of the simulation model (10) by a user and that preferably extends in arc-like form or hoop-like form from a first axial end section of the hollow cylindrical section (11) to a second axial end section of the hollow cylindrical section (11).

6. A simulation model (10) in accordance with one of the preceding claims, **characterized in that** the simulation model (10) is produced from a material, preferably from metal and/or plastic, that is opaque for light introduced into the hollow cylindrical section (11) and for environmental light.

7. A blood treatment machine having a simulation model (10) in accordance with one of the preceding claims and a blood leak detector (1) that has at least one light detector, **characterized in that** the at least one opening (13, 14) in the radial outer side of the hollow cylindrical section (11) of the simulation model (10) and the at least one light detector of the blood leak detector (1) are arranged such that light deflected by the light guidance element and introduced into the hollow cylindrical section (11) is conducted through the at least one opening (13, 14) to the at least one light detector.

8. A blood treatment machine in accordance with claim 7, **characterized in that** the simulation model (10) is releasably or non-releasably connected to the blood treatment machine.

9. A blood treatment machine in accordance with claim 7 or claim 8, **characterized in that** the blood treatment machine comprises a control apparatus that is configured to control or regulate the light sources of a simulation model (10) in accordance with one of the claims 1 to 6.

10. A blood treatment machine in accordance with claim 9, **characterized in that** the control apparatus is further configured to synchronize a frequency of an illumination cycle of the light sources of the simulation model (10) in accordance with one of the claims 1 to 6 with a recording frequency or measurement frequency of the blood leak detector (1).

11. A method of checking the function of a blood leak detector (1) of a blood treatment machine comprising the step: arranging a simulation model (10) in accordance with one of the claims 1 to 6 at the blood leak detector (1), preferably arranging the hollow cylindrical section (11) of the simulation model (10) in a tube receiver (2) of the blood leak detector (1).

12. A method in accordance with claim 11, further comprising the step: introducing light having a predetermined red portion and/or green portion into the hollow cylindrical section (11) of the simulation model (10) by means of the at least one light source, with the red portion and/or green portion of the light introduced by the light source being set such that it corresponds to the red portion and/or green portion of light that is reflected when, while either no fluid or a specific fluid flows in a tube arranged in the tube receiver (2), light of a predetermined wavelength is transmitted in or through the tube and/or the fluid.

13. A method in accordance with claim 12, **characterized in that** the red portion and/or the green portion of the light introduced by the light source is set such that it corresponds to the red portion and/or the green portion of light that is reflected when, while saline solution or plasma or dialysis fluid or plasma having a specific blood portion flows in the tube arranged in the tube receiver (2) and light of a specific wavelength is transmitted in or through the tube and/or the fluid.

**Revendications**

1. Modèle de simulation (10) permettant de simuler un tuyau flexible, comportant au moins une partie cylindrique creuse (11) qui est conçue pour être disposée dans un logement pour tuyau (2) d'un détecteur de fuite de sang (1), de préférence un détecteur de fuite de sang (1) d'une machine de traitement du sang, un logement (12) pour une source de lumière respective étant prévu à chaque extrémité axiale de la partie cylindrique creuse (11), logement au moyen duquel de la lumière peut être introduite dans la partie cylindrique creuse (11), et au moins un élément de guidage de la lumière qui est disposé dans la partie cylindrique creuse (11) et conçu pour dévier de la lumière introduite dans la partie cylindrique creuse (11) de telle manière que la lumière sort de la partie cylindrique creuse (11) par au moins une ouverture (13, 14) située dans un côté extérieur radial de la partie cylindrique creuse (11), **caractérisé en ce qu'**une source de lumière respective est disposée dans les logements (12) situés aux extrémités axiales de la partie cylindrique creuse (11), ladite source de lumière introduisant de préférence de la lumière rouge et/ou verte dans la partie cylindrique creuse (11).

2. Modèle de simulation (10) selon la revendication 1, **caractérisé en ce que** l'élément de guidage de la lumière est un miroir (16) qui est disposé de manière à dévier de sensiblement 90° la lumière introduite.

3. Modèle de simulation (10) selon l'une des revendications précédentes, **caractérisé en ce que**, dans le côté extérieur radial de la partie cylindrique creuse (11), sont prévues deux ouvertures (13, 14) de préférence opposées, entre lesquelles est de préférence disposé l'au moins un élément de guidage de la lumière.

4. Modèle de simulation (10) selon la revendication 1, **caractérisé en ce que** la source de lumière est une DEL (7, 8) qui est ancrée fixement dans le logement (12), de préférence par ajustement forcé et/ou collage.

5. Modèle de simulation (10) selon l'une des revendications précédentes, comportant en outre une partie formant poignée (15) qui est prévue pour faciliter la saisie du modèle de simulation (10) par un utilisateur et qui s'étend de préférence en forme d'arc ou en forme d'étrier d'une première partie d'extrémité axiale de la partie cylindrique creuse (11) à une seconde partie d'extrémité axiale de la partie cylindrique creuse (11).

6. Modèle de simulation (10) selon l'une des revendications précédentes, **caractérisé en ce que** le modèle de simulation (10) est fabriqué en un matériau opaque pour la lumière introduite dans la partie cylindrique creuse (11) et pour la lumière ambiante, de préférence en métal et/ou en matière plastique.

7. Machine de traitement du sang comportant un modèle de simulation (10) selon l'une des revendications précédentes et un détecteur de fuite de sang (1) qui présente au moins un détecteur de lumière, **caractérisée en ce que** l'au moins une ouverture (13, 14) située dans le côté extérieur radial de la partie cylindrique creuse (11) du modèle de simulation (10) et l'au moins un détecteur de lumière du détecteur de fuite de sang (1) sont disposés de telle manière que de la lumière introduite dans la partie cylindrique creuse (11) et déviée par l'élément de guidage de la lumière est guidée par l'au moins une ouverture (13, 14) jusqu'à l'au moins un détecteur de lumière.

8. Machine de traitement du sang selon la revendication 7, **caractérisée en ce que** le modèle de simulation (10) est relié de manière libérable ou non libérable à la machine de traitement du sang.

9. Machine de traitement du sang selon la revendication 7 ou 8, **caractérisée en ce que** la machine de traitement du sang présente un dispositif de commande qui est conçu pour commander ou réguler les sources de lumière d'un modèle de simulation (10) selon l'une des revendications 1 à 6.

10. Machine de traitement du sang selon la revendication 9, **caractérisée en ce que** le dispositif de commande est en outre conçu pour synchroniser une fréquence du cycle d'éclairage des sources de lumière du modèle de simulation (10) selon l'une des revendications 1 à 6 avec la fréquence d'entrée ou la fréquence de mesure du détecteur de fuite de sang (1).

11. Procédé de vérification du fonctionnement d'un détecteur de fuite de sang (1) d'une machine de traitement du sang, comportant l'étape consistant à : disposer un modèle de simulation (10) selon l'une des revendications 1 à 6 sur le détecteur de fuite de sang (1), de préférence disposer la partie cylindrique creuse (11) du modèle de simulation (10) dans un logement pour tuyau (2) du détecteur de fuite de sang (1).

12. Procédé selon la revendication 11, comportant en outre l'étape consistant à : introduire de la lumière avec une teneur en rouge et/ou en vert prédéfinie dans la partie cylindrique creuse (11) du modèle de simulation (10) au moyen d'au moins une source de lumière, la teneur en rouge et/ou en vert de la lumière introduite par la source de lumière étant réglée de manière à correspondre à la teneur en rouge et/ou en vert de la lumière qui est réfléchie quand, tandis qu'aucun fluide ou qu'un fluide donné circule dans un tuyau disposé dans le logement pour tuyau (2), de la lumière d'une longueur d'onde prédéfinie est envoyée dans ou à travers le tuyau et/ou le fluide.

13. Procédé selon la revendication 12, **caractérisé en ce que** la teneur en rouge et/ou en vert de la lumière introduite par la source de lumière est réglée de manière à correspondre à la teneur en rouge et/ou en vert de la lumière qui est réfléchie quand, tandis que de la solution saline ou du plasma ou du fluide de dialyse ou du plasma avec une teneur en sang donnée circule dans le tuyau disposé dans le logement pour tuyau (2), de la lumière d'une longueur d'onde prédéfinie est envoyée dans ou à travers le tuyau et/ou le fluide.

Fig. 1

Fig. 2

Fig. 3

Öffnung für
LED

12

15

11

13

Fenster
rechts

Fenster
links

14

Öffnung für LED

12

Fig. 4

Fig. 5

Fig. 6

EP 3 921 618 B1

Fig. 7

15

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102017001484 A1
- US 5601080 A